# EUROPEAN PATENT SPECIFICATION

(11) **EP 3 820 449 B1**
(45) Date of publication and mention of the grant of the patent: **14.05.2025**
(21) Application number: 19736749.3
(22) Date of filing: 11.07.2019
(51) Int. Cl.: A61K 9/08, A61K 47/44, A61K 31/00, A61K 31/575

(54) **COMPOSITION CONTAINING A 7BETA-HYDROXYCHOLESTEROL AND A LIPID VEHICLE, AND ITS USE IN THE TREATMENT OF NEOPLASTIC PATHOLOGIES**
ZUSAMMENSETZUNG, DIE EIN DERIVAT VON 7BETA-HYDROXYCHOLESTEROL UND EINEN LIPIDTRÄGER ENTHÄLT, UND IHRE VERWENDUNG ZUR BEHANDLUNG VON NEOPLASIEN
COMPOSITION CONTENANT UN 7 BÊTA-HYDROXYCHOLESTÉROL ET UN VÉHICULE LIPIDIQUE, ET SON UTILISATION DANS LE TRAITEMENT DE PATHOLOGIES NÉOPLASIQUES

(30) Priority: 11.07.2018 EP 18305933
(43) Date of publication of application: 19.05.2021
(73) Proprietor: Beta Innov, 75015 Paris (FR)
(72) Inventor: MERSEL, Marcel, 34090 Montpellier (FR); RAKOTOARIVELO, Clovis, 34070 Montpellier (FR)
(74) Representative: Santarelli
(86) International application number: PCT/EP2019/068670
(87) International publication number: WO 2020/011916

(56) References cited:
- WO-A1-2013/168096
- FR-A1- 3 013 048

## Description

The invention relates to a composition comprising a 7beta-hydroxycholesterol derivative and a lipid vehicle, the method of preparation thereof and said composition for use in the treatment of neoplastic pathologies, in particular glioblastoma multiforme.

Said lipid vehicle comprises an oil or a mixture of oils, advantageously a vegetable oil or a mixture of vegetable oils.

In particular, the invention relates to a composition, as defined in claim 1, comprising a 7beta-hydroxycholesterol derivative and a lipid vehicle, wherein said lipid vehicle comprises an oil or a mixture of oils, excluding a lipid vehicle consisting of vesicles formed of one or more lipid layer(s), such as for example vesicles of the liposome or micelle type.

Advantageously, said composition is in liquid form and can be administered by the oral route.

In particular, said 7beta-hydroxycholesterol derivative is in solution in said lipid vehicle.

In the present description, "7beta-hydroxycholesterol" or "7β-hydroxycholesterol" will be equally used.

Glioblastoma multiforme (GBM), or grade IV astrocytoma, is a brain tumour characterized essentially by the transformation of glial cells, which include astrocytes, into cancer cells.

Despite the substantial scientific and therapeutic advances in oncology, such as in the fields of immunotherapy and nanotechnology, GBM is still an incurable cancer. At best, researchers and doctors are satisfied when median patient survival can be prolonged by at most fifteen months.

Major problems posed by treatment of GBM are: (a) relapse caused by stem cells. In fact, when the existing therapies succeed in eradicating all or part of the tumour, stem cells are often responsible for tumour recurrence; (b) the molecules used in chemotherapy do not easily cross the blood-tumour barrier (BTB) and therefore low quantities of the anti-GBM agent reach the tumour site. The use of mini-pumps (Alzet^{®}), reservoirs (Mamiya^{®}) or supports (Gliadel^{®}) implanted in the GBM tumour in order to circumvent the problem posed by the BTB does not significantly improve the efficacy of chemotherapy. The effect of irradiation of the brain with ultrasound, to make the BTB flexible, on the efficacy of chemotherapy is currently being studied in animals.

In chemotherapy, one of the leading treatments is a combination therapy, which consists of administering Avastin^{®} (inhibition of binding of VEGF to its receptors) and irinotecan^{®} (inhibitor of topoisomerase I). Triple therapy of the PVC type (Procarbazine, DNA alkylating agent; Vincristine, inhibition of microtubule polymerization; CCNU, non-specific alkylating agent) is currently very controversial. Temozolomide, an alkylating agent of guanine, combined with radiotherapy, shows an increase in median survival by 2 to 3 months for patients who have hypermethylated DNA (Stupp protocol). Clinical trials using the TumorTreating Fields (TTF) approach as a supplement to the Stupp protocol are in progress. Clinical trials (phase III) testing cilengitide (inhibition of some integrin receptors) and talampanel (blocking the glutamate channels of the AMPA type) are in progress.

Also, chemotherapies showing efficacy for cancers other than GBM do not improve the median survival for patients with GBM. There may be mentioned for example bleomycin (administered for Hodgkin's lymphoma), afatinib dimaleate and cisplatin (administered for non-small-cell lung cancer) and cyclophosphamide (administered for breast cancer).

Application WO2013/168096 describes a family of 7β-hydroxycholesterol derivatives showing antitumour efficacy on human cancer cells *in vitro,* including GBM, in particular in liposome form or in the form of ethanol solution. In particular, the compound prepared in Example 6 of this application (called "compound BIM2b" in the examples in the present application) has shown activity *in vitro* on a human GBM line, the U87-MG line (U 87).

FR3013048 relates to 7β-hydroxycholesterol derivatives which are not esterified in C3-OH, useful as anti-tumoral agents.

There is therefore a need for a formulation allowing said 7β-hydroxycholesterol derivative, namely a molecule having therapeutic activity, to cross the obstacle of the blood-tumour barrier (BTB), in order to reach, in particular, a tumour located in the brain, while containing a sufficient quantity of this therapeutic molecule.

A formulation is also sought that allows easy administration, preferably by the oral route, and that does not cause toxic effects over a treatment time that may reach several weeks, or even several months, taking into account the patients' precarious state of health.

The steroidal compounds, such as steroid hormones, mineralocorticoids and glucocorticoids lack the side chain that forms part of cholesterol and of the oxysterol family. Also, in certain steroidal compounds, there are ketone functions combined with alcohol functions and aromatic rings, which is not the case for cholesterol and the commonest oxysterols.

Accordingly, steroidal compounds usually dissolve more easily in oils than cholesterol and 7β-hydroxycholesterol derivatives, in particular the compounds of formula (I) below, for the following reasons:
- the steric hindrance of the steroidal compounds is less than that of cholesterol and the 7β-hydroxycholesterol derivatives mentioned above,
- the steroidal compounds have more chemical functions and rings that have delocalized electrons than cholesterol and 7β-hydroxycholesterol derivatives, and
- the chemical functions and cyclic structures with polar characters found in the steroidal compounds allow binding to the acid functions, alcohols, double bonds and certain rings (spinasterol, schottenol) present in oil and therefore better dissolution in oil than cholesterol and the 7β-hydroxycholesterol derivatives of formula (I) below.

It has now been found that a formulation combining a 7β-hydroxycholesterol derivative and a lipid vehicle, wherein said lipid vehicle comprises an oil or a mixture of oils, excluding a lipid vehicle consisting of lipid vesicles formed of one or more lipid layer(s), allowed this sterol derivative to cross the BTB, as well as obtaining remarkable anti-GBM activity *in vivo* in animals.

Preferably, said formulation combines a 7β-hydroxycholesterol derivative and a lipid vehicle, wherein said lipid vehicle comprises an oil or a mixture of oils, excluding a lipid vehicle consisting of lipid vesicles formed of one or more lipid layer(s) or consisting of at least one phospholipid in the non-liposomal form.

Advantageously, said 7β-hydroxycholesterol derivative is in solution in the lipid vehicle according to the invention.

Obtaining a combination of these properties, namely the crossing of the BTB, the administration of active product in a sufficient amount, the absence of toxicity and the obtention of a therapeutical effect *in vivo* is particularly difficult to achieve, all the more in the field of neoplastic pathologies, where crossing the BTB alone is a major obstacle to the efficiency of the active product.

According to the invention, said lipid vehicle comprising an oil or a mixture of oils is, in particular, a pharmaceutically acceptable lipid vehicle.

In particular, said lipid vehicle comprises saturated and/or mono-unsaturated and/or poly-unsaturated fatty acids, or their mixtures, in particular in the form of mono-, di- or tri-glycerides, or their mixtures.

By "lipid vesicle formed of one or more lipid layer(s)" is meant a lipid vesicle comprising or constituted by one or more lipid layer(s), such as for example the vesicles of the liposome or micelle type.

Advantageously, the lipid vehicle according to the invention comprises at least one lipid which is not a phospholipid, said phospholipid being in the liposomal or non-liposomal form.

Advantageously, it was also found that this formulation is stable at a pH of about 1.5 (pH of gastric digestion in humans) and at a pH of about 8.5 to 10 (pH of digestion at the level of the Vater zone in humans), which is particularly sought for allowing the active compound to reach the site of action, in the context of administration by the oral route.

The invention therefore relates, according to a first aspect, to a composition comprising at least one 7β-hydroxycholesterol derivative and a lipid vehicle, wherein said lipid vehicle comprises an oil or a mixture of oils, excluding a lipid vehicle consisting of lipid vesicles formed of one or more lipid layer(s) or consisting of phospholipids in the non-liposomal form, in which said 7β-hydroxycholesterol derivative corresponds to formula (I) 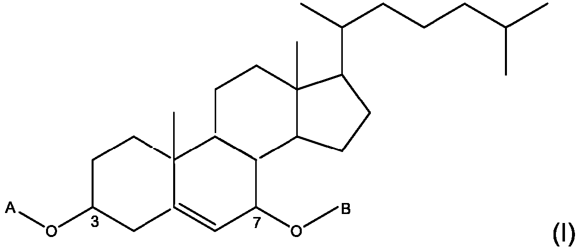 in which:
- A represents
   a -C(O)R₁ group in which R₁ is a saturated heterocycle comprising 5 to 14 members and including 1 or 2 heteroatoms, unsubstituted or substituted with at least one linear or branched C₁-C₆ alkyl or a group selected from OR, NRR', NHR and SR, where R and R', independently, represent hydrogen, a linear or branched C₁-C₁₂, preferably C₁-C₆, alkyl, or an unsubstituted aryl;
   or a -(R₂)ₙ- group in which R₂ is an amino acid residue bound by its C-terminal end and n= 1 to 3, each of the R₂ being identical or different, in which the N-terminal end of said amino acid may be substituted with a -C(O)R₃ group in which R₃ is a mono- or polycyclic C₆-C₁₄ arylalkyl group; a mono- or polycyclic C₅-C₁₄ heteroarylalkyl group that may comprise one or more heteroatoms, which may be identical or different; a mono- or polycyclic C₆-C₁₄ arylalkyloxy group or a mono- or polycyclic C₅-C₁₄ heteroarylalkyloxy group that may comprise one or more heteroatoms, which may be identical or different,
- B represents a -C(O)R₄ group, in which R₄ is a linear or branched C₁-C₁₂, preferably C₁-C₆, alkyl, unsubstituted or substituted with a group selected from OR, NRR', NHR and SR, as defined above; an aryl group, unsubstituted or substituted with a group selected from OR, NRR', NHR and SR, as defined above; or R₄ represents OR₅, in which R₅ is a linear or branched C₁-C₁₂, preferably C₁-C₆, alkyl.

The alkyl group denotes a linear or branched C₁-C₁₂ group such as the methyl, ethyl, propyl, isopropyl, butyl, isobutyl, sec-butyl, tert-butyl, pentyl, isopentyl, sec-pentyl, tert-pentyl, neo-pentyl, hexyl, isohexyl, sec-hexyl, tert-hexyl, heptyl, octyl, nonyl, decyl, undecyl or dodecyl groups, the linear or branched C₁-C₆ alkyl groups being preferred.

The aryl group denotes an unsaturated, monocyclic or polycyclic, carbocyclic, C₆-C₁₄ group, such as the phenyl, naphthyl, indenyl, anthracenyl groups and more particularly the phenyl group.

By "heteroatom" is meant an oxygen, nitrogen or sulphur atom.

Advantageous amino acid residues are, for example, methionyl, glycinyl or alanyl units.

Preparation of the compounds of formula (I) is described in applications EP2666382 and WO2013/168096.

"Comprising" can be understood, within the meaning of the present description, as encompassing the meanings "containing", "constituted by" or "consisting of".

"Constituted" by" or "consisting of", within the meaning of the present description, excludes the presence of other features apart from the ones following said wording.

Advantageously, said 7β-hydroxycholesterol derivative is in solution in said lipid vehicle.

The lipid vehicle comprises an oil or a mixture of oils, advantageously a vegetable oil or a mixture of vegetable oils.

In particular, said lipid vehicle is constituted by an oil or a mixture of oils, advantageously of a vegetable oil or a mixture of vegetable oils.

In particular, said 7beta-hydroxycholesterol derivative is in solution in said oil or mixture of oils, preferably a vegetable oil or mixture of vegetable oils, advantageously totally dissolved in said oil or a mixture of oils, preferably in said vegetable oil or mixture of vegetable oils.

By "vegetable oil" is meant a fatty substance extracted from an oleaginous plant, i.e. a plant certain parts of which such as the seeds, nuts or fruits contain lipids, in particular an edible oil.

Vegetable oil comprises saturated, mono-unsaturated and/or poly-unsaturated fatty acids of vegetable origin, which are mostly present in the form of acylglycerols, also named glycerides (esters of glycerol and fatty acids). Said acylglycerols are the major constituents of the oil, in particular, in an amount of at least 80%, preferably at least 90%, or else at least 95% of said vegetable oil. Said acylglycerols can be in the form of mono-, di- or tri-glycerides, or their mixtures.

Preferably, said fatty acids of vegetable origin comprise from 4 to 24 carbon atoms (C4 to C24), in particular 10, 12, 14, 16, 18, 20 or 22 carbon atoms. Said fatty acids can comprise, for example, from one to three double bonds in the carbon chain.

Said vegetable oil can also comprise, for example, minor amounts, such as less than 20%, preferably less than 10%, or else less than 5% of compounds other than acylglycerols, such as, for example, phospholipids, phytosterols, tocopherols, sphyngolipids, carotenoids etc.

Said vegetable oil may be selected, for example, from argan oil, avocado oil, linseed oil, sunflower oil, palm oil, cabbage palm oil, coconut oil, grapeseed oil, black mustard oil, poppy seed oil, shea butter oil, sweet almond oil, soya oil, peanut oil, cottonseed oil, sesame oil, olive oil, maize oil, cocoa oil, castor oil, Moringa oil (or Ben oil), rapeseed oil, annatto oil, wheatgerm oil, safflower oil, walnut oil, hazelnut oil, turnip rape oil or mixtures thereof.

A vegetable oil with low vitamin E content, in particular argan oil, will preferably be used.

Vegetable oil may also be synthetically produced by synthesis, for example, by preparing oils having similar chemical composition, for example preparing oils including glycerides.

The use of an oil or a mixture of oils, advantageously a vegetable oil or a mixture of vegetable oils, as the lipid vehicle in the composition, makes it possible to obtain a composition that may contain a sufficient quantity of compound of formula (I) to obtain both passage through the blood-tumour barrier (BTB) and the required therapeutic activity.

Said oils, in particular the vegetable oils, are preferably used in a pharmaceutically acceptable form.

Moreover, the formulations according to the invention comprising such a lipid vehicle have very good stability at extremes of pH such as a pH of about 1.5 (pH of gastric digestion in humans) and at a pH of about 8.5 to 10 (pH of digestion at the level of the Vater zone in humans), which makes it possible to preserve the activity of the compound of formula (I). Such properties have not been demonstrated in the case of lipid vesicles formed of one or more lipid layer(s), such as for example the vesicles of the liposome or micelle type.

Preferred compounds of formula (I) are those in which:
- A represents a -C(O)R₁ group in which R₁ is a saturated heterocycle comprising 5 to 14 members and including 1 or 2 heteroatoms, unsubstituted or substituted with at least one linear or branched C₁-C₆ alkyl;
- B represents a -C(O)R₄ group, in which R₄ is a linear or branched C₁-C₁₂, preferably C₁-C₆, alkyl or R₄ represents OR₅, in which R₅ is a linear or branched C₁-C₁₂, preferably C₁-C₆, alkyl.

According to a preferred aspect, R₁ is a saturated heterocycle comprising 5 members and including 2 oxygen atoms, substituted with at least one linear or branched C₁-C₆ alkyl, in particular a methyl group. In particular R₁ is a 2,2-dimethyl-1,3-dioxolane group.

Preferably, R₄ is a linear or branched C₁-C₆ alkyl, in particular a methyl group.

According to a preferred aspect, B represents an acyl group in which the alkyl group is C₁-C₆, in particular acetyl or an alkoxycarbonyl group in which the alkyl group is C₁-C₆, in particular a tert-butoxycarbonyl group.

Other preferred compounds of formula (I) are those in which:
- A represents a -(R₂)ₙ- group in which R₂ is an amino acid residue and n = 2; or
- A represents a -(R₂)ₙ- group in which R₂ is an amino acid residue, n = 2 and the N-terminal end of said amino acid is substituted with an arylalkoxycarbonyl group, in particular benzyloxycarbonyl; or
- A represents an alanyl radical bound to a glycinyl radical, optionally substituted at its N-terminal end with an arylalkoxycarbonyl group, in particular benzyloxycarbonyl; or
- A represents a methionyl radical bound to a glycinyl radical, optionally substituted at its N-terminal end with an arylalkoxycarbonyl group, in particular benzyloxycarbonyl, and
- B is as defined above, in its general or preferred definitions.

Preferred compounds of formula (I) are as follows:
- 7-((*tert*-butoxycarbonyl)oxy)-10,13-dimethyl-17-(6-methylheptan-2-yl)-2,3,4,7,8,9,10,11,12,13,14,15,16,17-tetradecahydro-1*H-*cyclopenta[a]phenanthren-3-yl 2-(2-(((benzyloxy)carbonyl)amino)-acetamido)propionate, also given the simplified name "3-benzyloxycarbonyl-glycinyl-alanyl-7-β-O-tert-butyloxycarbonyl-cholesterol" or compound BIM1a;
- 7-acetoxy-10,13-dimethyl-17-(6-methylheptan-2-yl)-2,3,4,7,8,9,10,11,12,13,14,15,16,17-tetradecahydro-1H-cyclopenta[a]phenanthren-3-yl 2-(2-(((benzyloxy)carbonyl)amino)-acetamido)propionate, also given the simplified name "3-benzyloxycarbonyl-glycinyl-alanyl-7-β-O-acetyl-cholesterol" or compound BIM1b;
- 7-((tert-butoxycarbonyl)oxy)-10,13-dimethyl-17-(6-methylheptan-2-yl)-2,3,4,7,8,9,10,11,12,13,14,15,16,17-tetradecahydro-1H-cyclopenta[a]phenanthren-3-yl 2,2-dimethyl-1,3-dioxolane-4-carboxylate, also given the simplified name "3-(*S*)-2,2-dimethyl-1,3-dioxolane-4-carboxyl-7-β-O-tert-butyloxycarbonyl-cholesterol" or compound BIM2a;
- 7-acetoxy-10,13-dimethyl-17-(6-methylheptan-2-yl)-2,3,4,7,8,9,10,11,12,13,14,15,16,17-tetradecahydro-1H-cyclopenta[a]phenanthren-3-yl 2,2-dimethyl-1,3-dioxolane-4-carboxylate also given the simplified name "3-(*S*)-2,2-dimethyl-1,3-dioxolane-4-carboxyl-7-β-O-acetyl-cholesterol", or "cholest-5-ene-3,7-diol, 7-acetate 3-[[(4S)-2,2-dimethyl-1,3-dioxolan-4-yl]carboxylate], (3β,7β)" or compound BIM2b.

These compounds of formula (I) are described, respectively, in Examples 3, 4, 5 and 6 of application WO2013/168096.

A compound of formula (I) particularly preferred for the purposes of the invention is the "compound BIM2b" mentioned above, i.e. a compound of formula (I) in which A is a -C(O)R₁ group in which R₁ is a 2,2-dimethyl-1,3-dioxolane group and B is an acetyl group.

This compound corresponds to the formula

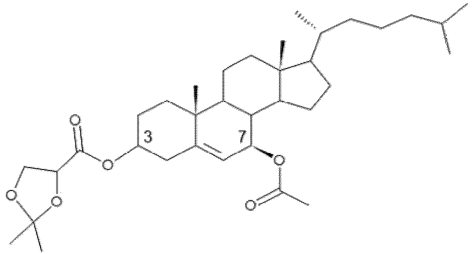

The compound BIM1a is a compound of formula (I) in which A is a 3-benzyloxycarbonyl-glycinyl-alanyl group and B is a tert-butoxycarbonyl group.

The compound BIM1b is a compound of formula (I) in which A is a 3-benzyloxycarbonyl-glycinyl-alanyl group and B is an acetyl group.

The compound BIM2a is a compound of formula (I) in which A is a-C(O)R₁ group in which R₁ is a 2,2-dimethyl-1,3-dioxolane group and B is a tert-butoxycarbonyl group.

In the present description, and unless stipulated otherwise, the ranges of values indicated are inclusive.

The content of compound of formula (I) in the composition according to the invention is preferably from 0.1 to 3.5% (w/v) of the total volume of the composition, preferably 3%. Preferably, said composition comprises the compound of formula (I) at a rate from 1 to 35 mg/ml, preferably 30 mg/ml.

The content of lipid vehicle, wherein said lipid vehicle comprises an oil or a mixture of oils, in particular of vegetable oil, in the composition according to the invention is preferably from 90 to 99%, in particular from 95 to 99% (v/v) of the total volume of the composition.

The compositions according to the invention may also contain at least one usual excipient or additive in the case of an oily composition, for example a co-solvent, such as for example an alcohol, or an antioxidant.

Said co-solvent, such as for example an alcohol, may be present in the composition for example at a rate of 0 to 5%, in particular 1 to 5% (v/v) of the total volume of the composition.

As alcohol, an alcohol of formula R-OH will preferably be used, in which R represents a C₂-C₆ hydrocarbon group, preferably ethanol.

The antioxidant may be selected from the compounds that are usual in this field, for example:
- a hydroxylated substance such as citric acid and derivatives thereof, in particular sodium citrate, calcium citrate or potassium citrate; ascorbic acid and derivatives thereof, in particular isoascorbic acid, sodium ascorbate or calcium ascorbate;
- a thiol derivative such as for example cysteine, acetylcysteine, dithiothreitol;
- an ethylenically unsaturated substance, such as sorbic acid, etc.

Said antioxidant may be present in the composition for example at a rate of 0.005 to 0.020% (w/v), in particular of 0.005 to 0.015% (w/v), preferably 0.01%.

A preferred antioxidant is selected from ascorbic acid and derivatives thereof.

A preferred composition according to the invention comprises 3% (w/v) of 7β-hydroxycholesterol derivative of formula (I), in particular of one of the preferred compounds of formula (I) mentioned above, and more particularly of compound BIM2b, 0.01% of anhydrous citric acid (w/v) and 1% of ethanol (v/v), in argan oil. Said composition may also be ethanol-free.

The invention also relates to a pharmaceutical composition comprising a composition as described above.

Said pharmaceutical composition may also contain another active ingredient soluble in a lipid medium, such as for example an anti-inflammatory agent, in particular a corticoid, or an antiepileptic agent.

The pharmaceutical compositions according to the invention may preferably be in a form suitable for administration by the oral route, in particular in liquid form, such as for example oral solution, oral suspension, drops, etc., or in encapsulated form, such as a capsule encapsulating said liquid formulation, in particular a hard or soft capsule based on gelatin.

The invention also relates to a method for preparing a composition comprising a 7β-hydroxycholesterol derivative and a lipid vehicle as described above, comprising the following steps:
- preparing a mixture containing the 7β-hydroxycholesterol derivative of formula (I) as described above, a lipid vehicle and a co-solvent,
- optionally adding an antioxidant, and
- if applicable, evaporating the co-solvent.

The lipid vehicle, the antioxidant and the co-solvent are as defined above. The preferred aspects mentioned above also apply to the method according to the invention.

In particular, said lipid vehicle consists of an oil or a mixture of oils, advantageously a vegetable oil or a mixture of vegetable oils, in particular argan oil.

Said oils, in particular the vegetable oils, are preferably used in a pharmaceutically acceptable form.

Preferred conditions of said method are as follows:
- the mixture is prepared at a temperature of the order of 22 to 35°C, preferably 33°C;
- the content of compound of formula (I) in the mixture is from 0.1 to 3.5% (w/v);
- the initial content of co-solvent, preferably ethanol, in the mixture is of the order of 1 to 5%, preferably 2.5% (v/v);
- the content of antioxidant, preferably citric acid and derivatives thereof, in the mixture is from 0.005 à 0.020% (w/v), in particular of 0.005 to 0.15%, preferably 0.01% (w/v).

The mixture containing the 7β-hydroxycholesterol derivative of formula (I), a lipid vehicle and a co-solvent and optionally an antioxidant may be mixed, for example, using a rotary evaporator with stirring until there is dissolution of the compound of formula (I).

The mixture can be carried out, for example, by mixing the 7β-hydroxycholesterol derivative of formula (I) and a lipid vehicle, as defined above, then adding a co-solvent, and, optionally, an antioxidant.

The content of co-solvent, and in particular of alcohol (in particular ethanol) obtained after evaporation is compatible with the current regulations for pharmaceutical forms (ICH Q3C(R6) Regulations).

The invention also relates to a composition comprising a 7β-hydroxycholesterol derivative of formula (I) and a lipid vehicle, as defined above, for use in the treatment of a neoplastic pathology, in particular glioblastoma multiforme.

The invention also relates to a composition comprising a 7β-hydroxycholesterol derivative of formula (I) and a lipid vehicle, as defined above, for use in the treatment of a malignant haemopathy, in particular a malignant haemopathy of the myeloid type.

The invention also relates to a pharmaceutical composition comprising a composition comprising a 7β-hydroxycholesterol derivative of formula (I) and a lipid vehicle, as defined above, for use in the treatment of a neoplastic pathology, in particular glioblastoma multiforme, or in the treatment of a malignant haemopathy, in particular a malignant haemopathy of the myeloid type.

The general and preferred aspects mentioned above, in particular for the 7β-hydroxycholesterol derivative of formula (I) and the lipid vehicle, also apply to these uses and methods.

The dose administered may be for example from 3 to 5 mg/kg per day with a duration ranging for example from 84 to 105 days. However, the dose may be increased depending on the patient's condition and the stage of the disease up to 30 mg/kg/day and the duration of the treatment may be increased for example to 2 months.

The invention is illustrated by Examples 1-2 (preparation) and Examples 3 to 8 (results).

The method of solubilization of BIM2b, as described in detail in Examples 1 and 2, allowed the dissolution of BIM2b in oils, particularly argan oil. In an advantageous aspect, over a range from 1 to 500 ml of oil, the conditions of adding the BIM2b powder to the oil, of mixing the BIM2b/oil system and the surface area/volume ratio at which mixing was carried out led to complete solubilization of the BIM2b powder in the oil.
Figure 1 shows silica column chromatography of a lipid extract from a healthy mouse brain treated with the oily formulation of Example 2.
Figure 2 shows silica column chromatography of a lipid extract from the brain of a dog with glioblastoma treated with the oily formulation of Example 2.
Figure 3 shows monitoring the stability, by HPTLC, of the oily formulation of Example 2 after the tests of resistance to extremes of pH.
Figure 4 shows the total regression of the tumour in a dog subject with glioblastoma treated with the oily formulation of Example 2.

The following abbreviations are used:
7β-acetyl-CH: 7β-acetyl-cholesterol
7β-OHCH: 7β-hydroxycholesterol
APCI: Atmospheric Pressure Chemical Ionization source
TLC: thin-layer chromatography
CH: cholesterol
CHCl₃: chloroform
Compound BIM2b: compound 7-acetoxy-10,13-dimethyl-17-(6-methylheptan-2-yl)-2,3,4,7,8,9,10,11,12,13,14,15,16,17-tetradecahydro-1H-cyclopenta[a]phenanthren-3-yl 2,2-dimethyl-1,3-dioxolane-4-carboxylate, also called cholest-5-ene-3,7-diol, 7-acetate 3-[[(4S)-2,2-dimethyl-1,3-dioxolan-4-yl]carboxylate], (3β,7β)
Compound BIM2b PO: compound BIM2b in the form of oily formulation of Example 2 for administration by the oral route
EtOH: ethanol
Folch: CHCl₃:MetOH (2:1, v:v)
AO: argan oil
HCl: hydrochloric acid
GBM: glioblastoma multiforme
HPTLC: high-performance thin-layer chromatography
KCI: potassium chloride
MetOH: methanol
NaOH: sodium hydroxide
p: weight
MW: molecular weight
RT: ambient temperature
UPLC MS: ultrahigh-performance liquid chromatography-mass spectrometry
v: volume

### A/ MOLECULES AND VEHICLE USED AND ANALYSES

### 1) Active substance

Cholest-5-ene-3,7-diol, 7-acetate 3-[[(4S)-2,2-dimethyl-1,3-dioxolan-4-yl]carboxylate], (3β,7β) was synthesized at GLP/GMP grade as described in application WO2013/168096 (obtained in the form of powder as described in Example 6).

This molecule, the formula of which is given below, is called BIM2b throughout the experimental section. 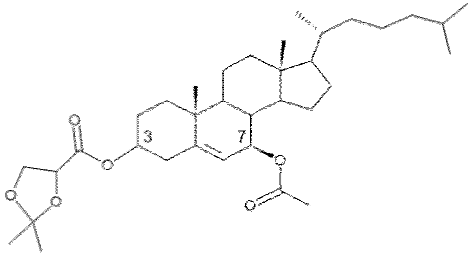 Molecular weight: 572.83

### 2) Degradation products and major metabolites of the active substance

### a) 7β-Acetyl-cholesterol was synthesized as described in application WO2013/168096 (compound 1.4).

This molecule, the formula of which is given below, is called 7β-acetyl-CH throughout the experimental section. 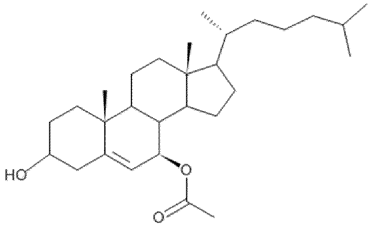 Molecular weight: 444

### b) 7β-Hydroxycholesterol was supplied by SIGMA (reference 700035P).

This molecule, the formula of which is given below, is called 7β-OHCH throughout the experimental section. 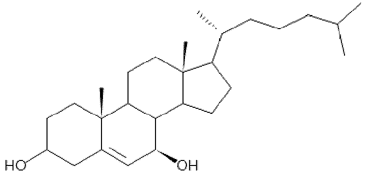 Molecular weight: 402.65

### c) The cholesterol was supplied by SIGMA (reference C8503).

This molecule, the formula of which is given below, is called CH throughout the experimental section. 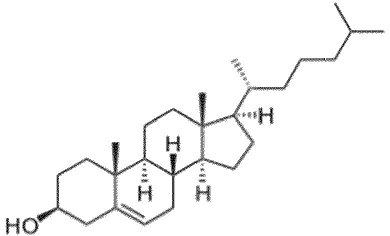 Molecular weight: 386.65

### d) The vehicle selected is argan oil (Olsana, France), called AO throughout the experimental section.

### B) MATERIALS AND METHODS

### I) MATERIALS

### I.1) Chromatography

### I.1.1) High-performance thin-layer chromatography (HPTLC) and adsorption chromatography on a conventional silica column

- Multi-plate tanks for chromatography 20 x 20 cm (reference 552-0359)
- Chromatography columns with glass frit (useful length: 300 mm; inside diameter = 11 mm; key-operated PTFE stopcock 1.5 mm) (Analytic Lab, reference LE-AT1811)
- Powdered silica gel 60 (0.063-0.200 mm) (reference 1.07734.1000)
- HPTLC plate: silica gel 60 F254 with concentration zone (2.5 cm) on aluminium foil (VWR, reference 1055830001).
- Nebulizer and Erlenmeyer flask 100 ml (Witeg, reference 2147100, NS 14.5/23)
- Hot plate (Stuart SB162)
- Borosilicate glass tube
- Scanner (Epson Perfection V370 Photo)
- Microcapillaries Drummond 10µl (Sigma, reference P1924)

### I.1.2) Chromatography on adsorption cartridge or reversed-phase

- SEP PAK Classic Silica (Waters, reference WAT051900)
- SEP PAK Plus C18 (Waters, reference WAT023635)

### I.1.3) Ultra high-performance liquid chromatography - Mass spectrometry (UPLC - MS)

Quantification of the molecules being investigated (BIM2b and its metabolites) was elaborated using a Nexera X2 UPLC system (Shimadzu) equipped with a Kinetex EVO C18 column (particle size: 1.9 µm; column dimensions: 100 x 2.1 mm, Phenomenex). APCI detection is performed with a quadrupole (LCMS 8050, Shimadzu).

### I.2) Formulation of BIM2b for administration by the oral route (BIM2b PO)

- Sartorius Quintix precision balance (Sartorius, reference QUINTIX 124-1S)
- 5 ml borosilicate glass tubes 12 x 75 mm (Kimble, reference 73500-1275)
- 25 ml borosilicate glass tubes Pyrex 18 x 180 mm (Dutscher, reference 090482)
- 5 ml beaker
- 1-litre reaction flask with ground-glass stopper (female, 29/32NS) (VWR, reference 201-1359)
- R-215 Buchi rotary evaporator
- Phase-contrast inverted light microscope Eclipse TS-100F trinocular Nikon (Dutscher, reference: 094501) coupled to a C-mount digital camera (NIKON, DCMC510)

### I.3) Quantification of the molecules under investigation (BIM2b and its metabolites): homogenization of brains of wild-type mice and of tumours obtained after necropsy of brains of dogs with GBM

- Homogenizer (Ika eurostar 20 digital)
- Potter-Elvehjem tissue grinder (capacity: 4 ml; length: 120 mm; outside diameter: 11 mm) (VWR, reference 432-0200)
- Spherical separating funnel, 250 ml with PTFE stopcock (Dutscher, reference 479036)
- Separating funnel, 50 ml with PTFE stopcock (Dutscher, reference 084118)

### I.4) Organic solvents and other consumables

- Silica for column chromatography, silica gel 60 (0.063-0.200 mm) (Merck, reference 1.07734.1000)
- CHCl₃, HPLC grade (Sigma, reference 528730)
- EtOH absolute (Sigma, reference SI-32221)
- MetOH chromasolv for HPLC ≥ 99.9% (SIGMA, reference 34860)
- N-Hexane for HPLC (Fisher Scientific, reference 1070361)
- Diethyl oxide, stabilized for HPLC (Analytic Lab, reference FI-10254914)
- Acetone (Sigma-Aldrich, reference 322201)
- Orthophosphoric acid 99% (Analytic Lab, reference U1B203102H)
- Copper acetate 98% (Sigma, reference 326755/
- Butylated hydroxytoluene (BHT; Sigma, W218405, reference W218405)
- Hydrochloric acid min. 37% (HCl, Thermofisher, reference M0643H)
- Soda (NaOH/Thermofisher, reference 04815)
- Ammonium carbonate (Sigma, reference 207861)
- Potassium chloride (Fisher Scientific, reference W2752)
- Formol or formaldehyde solution (36.5-38% in water) (Sigma, reference F8775)
- Ammonium acetate (Merck, reference C138315)

### II) Methods

### II.1) HPTLC chromatography

This analytical approach was used for quantifying BIM2b, its degradation products and its major metabolites in the medicament (BIM2b PO) and brains of wild-type mice or GBM tumours from dogs that had this pathology. The threshold of detection of BIM2b and of its degradation products (7β-acetyl-CH and 7β-OHCH), determined experimentally, is 0.5 µg.

This method was validated by UPLC - MS.

### - Treatment of the HPTLC plates

The multi-plate chromatography tanks are saturated with the eluent system, the CHCl₃:MetOH mixture (1:1; v:v), for 1 h. The HPTLC plates are placed in the tanks; elution is stopped when the eluent front reaches 1.5-2 cm from the top edge of the plates. Then they are dried at ambient temperature for 1 h. Next, these plates are activated at 110°C for 15 min. Once they have cooled, they are wrapped in aluminium foil and are stored away from the light and moisture.

### - Depositing the samples on the HPTLC plates, and chromatographic conditions

All the samples are solubilized in acetone and are deposited with Drummond capillaries (10 µl) on the concentration zone of the HPTLC plates. When the standard ranges of BIM2b, 7β-acetyl-CH and 7β-OHCH are used, they are prepared from ethanol stock solutions of 0.1 mg/ml for each reference molecule.

Chromatographic separation is carried out in two steps: a pre-elution step (3 times) with the CHCl₃:MetOH mixture (2:1, v/v) as the eluent system for the concentration zone, followed by a separation step (3 times) for the separation zone carried out with the hexane:ether mixture (3:7, v/v) as the eluent system in order to identify and quantify the sterols.

Before chromatography, the tanks are saturated with the respective eluent systems for concentration and for separation.

The plates are dried between each elution using a cold air stream from a hair dryer.

### - Visualization and quantification of the oxysterols

The oxysterols are visualized according to the Macala method (1). The Macala reagent consists of an aqueous solution at 8% (w/v) of orthophosphoric acid and 3% (w/v) of copper acetate. Following chromatography, the HPTLC plates are dried, sprayed with the Macala reagent and heated on a hot plate to 140°C. The oxysterols turn Prussian blue and the cholesterol turns mauve. The coloured spots are scanned and then quantified by Image J (version 1.50 g).

Quantification of the oxysterols and cholesterol by the HPTLC method was validated by UPLC - MS.

### II.2) UPLC - MS

### Experimental conditions for elution and detection:

### - Quantification of BIM2b and 7β-OHCH

Isocratic elution is performed with a mobile phase consisting of MetOH containing 5 mM of ammonium acetate.

### - Quantification of 7β-acetyl-CH:

Elution is performed with a linear binary gradient. Eluent A was 0.1% (v:v) of formic acid and eluent B was MetOH containing 0.1% of formic acid. Elution begins with a mixture (v:v) of 20% A and 80% B and ends with 100% of eluent B; the duration of the gradient is 3 min.

The volume of the injections is 10µl and detection is performed by APCI in positive mode.

### II.3) Extraction and purification of BIM2b and its degradation products

Except for the quantification of BIM2b, 7β-acetyl-CH and 7β-OHCH during stability tests on BIM2b in formulated BIM2b (BIM2b PO), where the samples of BIM2b PO are applied directly on the HPTLC plates, extraction and purification of the oxysterols is necessary for quantifying these molecules in BIM2b PO exposed to conditions of extremes of pH, in brains of wild-type mice tube-fed by the oral route with BIM2 PO or in GBM tumours obtained from necropsy of a dog with GBM that occurred spontaneously (GBM dog subject), treated by the oral route with BIM2b and which survived for 8 months (mean survival of a dog with GBM is 2-3 months despite chemotherapy treatments).

The oxysterols are extracted by the method recommended by Folch et al. (2). Extraction takes place in a separating funnel. Briefly, extraction is performed with a CHCl₃:MetOH mixture (2:1, v:v; Folch); for this method, the ratio of the volume of Folch to the volume of the aqueous sample is 19. Rinse and break the phase with 0.2 volume of the organic phase of aqueous solution containing KCI at 0.74%.

For the case of extraction of the oxysterols from brains of healthy mice treated by the oral route with BIM2b PO or from a GBM tumour of a dog also treated by the oral route with BIM2b PO, the organic phase rinsed with 0.74% KCl is rinsed once more with 0.4 volume of CHCl₃:MetOH:H₂O mixture (3:48:47; v:v:v; upper phase of Folch). The dried residue is then solubilized in a suitable solvent for the experimental method that follows the extraction step.

### II.4) Samples requiring extraction before separation and quantification of the oxysterols

### - Test of resistance of BIM2b PO to an extreme acid pH followed by incubation at basic pH

For these tests, BIM2b PO is incubated firstly in an acid bath at pH 1.5 and then in a basic bath at pH 8.5. These 2 values correspond respectively to the physiological pHs of gastric digestion and of digestion at the level of the Vater ampulla in humans.

The acid aqueous bath is prepared from 1M and 0.1M HCl stock solutions. The basic bath is an aqueous solution of ammonium carbonate at 25 mM.

A first beaker containing 4 ml of acid bath (pH 1.5) is heated to 37°C before starting the experiment; 2 ml of BIM2b PO is then gently deposited on the surface of the bath. The reaction mixture is stirred with a magnetic bar for 10 h. The stirring is switched off, and the reaction mixture is left to stand for 2 h for proper reforming of the oily upper phase. During the 2 h with the reaction mixture left to stand, the basic bath (pH 8.5) is prepared in a second beaker and heated to 37°C. A 1-ml sample is taken from the lipid phase of the acid bath and is deposited gently on the aqueous surface of the basic bath. Stirring is started, for 10 h of incubation. After stopping the stirring, the whole mixture (BIM2b PO and the basic bath) is subjected to Folch extraction without rewashing the organic phase with the Folch upper phase.

### - Extraction and quantification of the oxysterols and their metabolites from brains of healthy mice treated with BIM2b PO (oral route)

The mouse brains dried and stored invididually in cryotubes at 180°C in liquid nitrogen are left to return to ambient temperature. Each brain is weighed and put in the Potter; 4 ml of 0.74% of cold KCI is added and the rest of the procedure takes place on a bed of ice. Each brain is ground 6 times at 1000 rev/min, each cycle comprising 5 minutes of grinding and 5 minutes of rest. The oxysterols are extracted according to the Folch procedure described above (II.3.). The lipid residue is then subjected to adsorption chromatography on a silica column.

The silica (8 g) is prepared in 40 ml of the hexane:ether mixture (diethyl-oxide (3:7, v/v) and then poured into the appropriate columns. The height of the silica bed is 18 cm and its volume is 15 ml.

A suitable quantity of mouse brain extract is dissolved in 1 ml of the first eluent system and deposited on the silica bed. Elution takes place in 4 steps in order to concentrate the fractions containing the oxysterols and remove as far as possible the lipids contaminating the mouse brain, in particular cholesterol.

The elutions proceed as follows:
1st elution: hexane:ether (3:7, v:v)
2nd elution: acetone:EtOH (95:5, v:v)
3rd elution: acetone:EtOH (93:7, v:v)
4th elution: EtOH

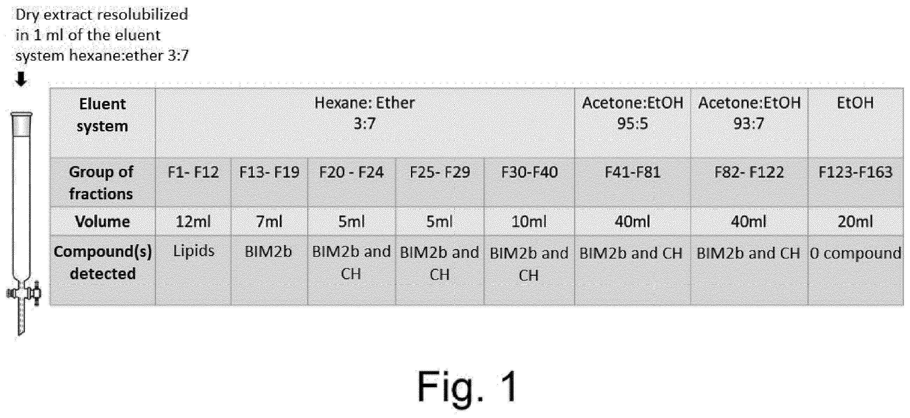

The elution procedure is described in Figure 1.

The groups of fractions are evaporated to dryness, dissolved in acetone and the appropriate volumes are subjected to HPTLC chromatography.

### - Extraction and quantification of BIM2b and its metabolites from a GBM tumour in a dog treated with BIM2b PO (oral route)

The GBM tumour was obtained by necropsy of a dog with glioblastoma, which was treated by the oral route with BIM2b PO and which lived with a good quality of life for 8 months. Necropsy was carried out 21 days after stopping the treatment with BIM2b PO. The tumour was fixed in formol mainly for histopathology analysis and a remaining fragment was analysed for quantification of BIM2b.

On fragments of brains of dogs without GBM and on a part of the tumour fragment, all the checks were carried out to demonstrate that fixation with formol did not alter the quality of the oxysterols or the yield in extraction thereof by the Folch technique.

The fragments of dog brains, stored individually in cryotubes at +4°C in a bath of formol, are dried on sopalin paper before they are weighed and put in the Potter. Just as for extraction of the mouse brains, each fragment was extracted after adding 4 ml of cold 0.74% KCI. The procedures for homogenization and extraction are the same as was described for the mouse brains in the Methods section.

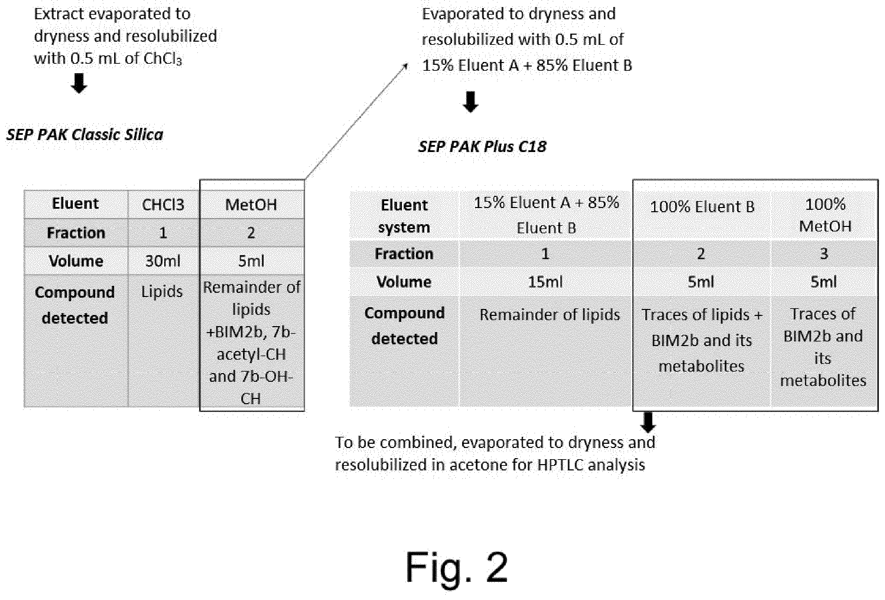

Dry lipid extract of the dog GBM tumour fragment was thus obtained and was chromatographed sequentially on a SEP PAK Classic Silica cartridge; then on SEP PAK Plus C18 (with eluent A = 5 mM of ammonium acetate/ultrapure water and B = 5 mM of ammonium acetate/MetOH). The chromatography process on SEP PAK columns is shown in Figure 2.

### C. Examples of preparation and results

### Example 1: Tests on lipid vehicles

The oils tested are argan oil, avocado oil, olive oil, peanut oil, and grapeseed oil. The tests were performed at the test tube scale.

Various quantities of BIM2b, ranging from 1 to 35 mg, were added to 1 ml of the oil selected, several times, to reach the quantity determined. The test tube is stirred gently between each addition of BIM2b.

The tubes are put in an incubator at 37°C for 24 h. During the first 5 hours of incubation, the tubes were brought out of the incubator every hour in order to stir them gently, and were put back in the incubator overnight. The next day, the appearance of the mixture of the oil and BIM2b was checked visually to determine the degree of dissolution of the powder in the oil. When the BIM2b seems to the naked eye to have dissolved completely in the oil, examination with the microscope is carried out to check for the presence or absence of BIM2b in the form of powder or crystals.

The results show that BIM2b dissolves completely in avocado oil, olive oil, peanut oil, and grapeseed oil at a concentration of 3 mg of BIM2b/ml of oil.

In argan oil (AO), BIM2b is dissolved up to a concentration of 30 mg (52µmol) of BIM2b/ml of AO.

### Example 2: Preparation of BIM2b PO

285 ml of argan oil (Olsana, France) was put in a 1L flask, used as the production container. The position of the flask is inclined and it is turned manually about its own axis in order to distribute the oil uniformly on the flask wall in an area/volume ratio: 1.6 cm⁻¹ (calculated result: 460 cm²/285 cm³). Maintaining this position, 9 g of BIM2b powder is incorporated over the whole wall so as to cover the entire surface of the flask. Next, 3 mL of ethanol containing 30 mg of citric acid is added (from a stock solution at 10 mg of citric acid/ml of ethanol); and then 12 ml of ethanol (i.e. 5% of ethanol). The flask is put on a Buchi^{®} R-215 angled rotary evaporator in a water bath at 30°C at a speed of 15 rpm for 2 h. To evaporate the ethanol, it is then left to evaporate in a water bath at 30°C overnight with the stopper open.

Thus, 300 ml of composition is obtained containing BIM2b in argan oil at a concentration of 30 mg/ml.

The BIM2b PO formulation thus obtained, which will be used in the examples given below, contains 3% (w/v) of BIM2b, 0.01% (w/v) of anhydrous citric acid and 1% of EtOH (v/v) (after evaporation) in argan oil.

Quantification of BIM2b in BIM2b PO and of its degradation products just after production of this batch shows a concentration of 30 mg of BIM2b/ml of BIM2b PO without any degradation products.

A study of stability of the BIM2b PO batches produced by this method shows, under the experimental conditions, the presence of 1% of degradation products (w/v) after storage for 6 months, and less than or equal to 2% (w/v) after storage of the same batch for 12 months.

### Example 3: Test of resistance of BIM2b PO to an acid pH followed by incubation at basic pH

The test procedure is described above in Section II) Methods.

After incubation of BIM2b PO in the successive acid (pH 1.5) and basic (pH 8.5) baths, the dry lipid extract was dissolved in acetone. Owing to lack of space (4 tracks at theoretical 15, 30, 40 and 60 µg), a standard range was not deposited.

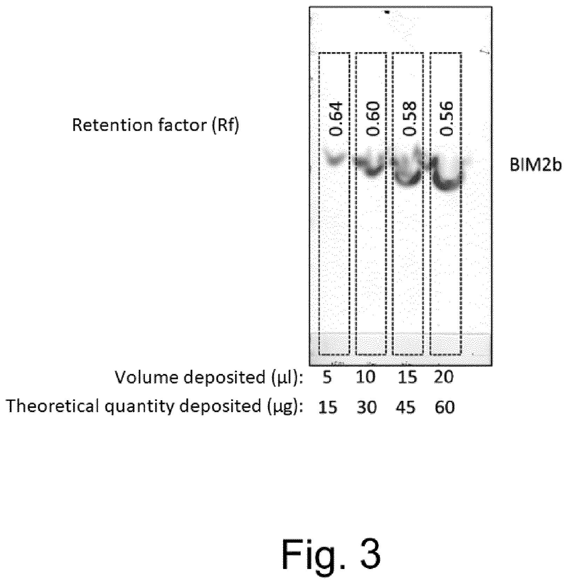

The analytical results from HPTLC presented in Figure 3 show detection of a single band, that of BIM2b, regardless of the quantity deposited, with a retention factor (Rf) from 0.5 to 0.64. The Rf values of the degradation products 7β-acetyl-CH and 7β-OHCH (not visible) determined experimentally are 0.37 and 0.24, respectively. These results show that BIM2b PO withstands conditions of extremes of pH without being degraded.

### Example 4: Investigation of toxicity of BIM2b PO in healthy mice

The composition from Example 2 was administered orally (by tube) to wild-type mice at a rate of 1.5 mg (50 µl of BIM2 PO) per animal, twice a day, 5 days out of 7 with a maximum duration of treatment of 21 days. In parallel, a control group of wild-type mice did not receive any treatment.

In the treated group, 2 mice were sacrificed after 18 days and 3 mice were sacrificed after 21 days. In the control group, 3 mice were sacrificed after 21 days.

The samples of internal organs (stomach, alimentary canal, liver, kidney, lung, heart) were immersed in 4% formaldehyde for 72 h and stored at 4°C, and then embedded in paraffin. The samples underwent macroscopic analysis before and after thinning.

Histological examination of the internal organs fixed with formaldehyde and toxicological quantifications were carried out on sections stained with haematoxylin-eosin-saffron according to a 4-level evaluation system complying with the nomenclature (3).

All the sections were examined for possible signs of cellular alteration, degeneration, necrosis, inflammation, fibrosis etc.

The conclusions are as follows (for each organ):
- Stomach: no lesion in any treated mouse
- Small intestine: minimal inflammation of a lymphoplasmacystic nature interpreted as forming part of the background noise. No significant lesion.
- Large intestine: no lesion in any of the mice.
- Liver: a tendency to reduction of cytoplasmic storage and hepatocellular hypertrophy were observed in the treated mice compared to the control mice. This change remained very diffuse and cannot be attributed with certainty to the treatment.
- Kidney: no significant lesion detected in any of the mice.
- Lung: minimal histiocytosis and slight alveolar inflammation in one of the treated mice, possibly caused by oral force-feeding.
- Heart: no lesion detected in any of the mice.

In conclusion, the results show that the molecule BIM2b does not entrain any toxicity. Administration of BIM2b at the indicated posology, twice a day, 5 times a week, with absence of treatment at the weekend, did not give rise to undesirable clinical effects. Likewise, the biological parameters investigated did not demonstrate hepatic or renal toxicity. Diabetes and hypercholesterolaemia, which might be expected with administration of a steroid, were not observed.

### Example 5: Study of tolerance of BIM2b PO in healthy dogs

The composition from Example 2 was administered orally to 3 beagle dogs with an average weight of 11 kg, at a rate of 15 mg (0.5 ml), twice a day (in the morning after the day's meal and in the evening while fasting), 5 days out of 7 with a duration of treatment of 21 days.

Calculation of the dose administered was based on the value specified for administration, of 3 mg/kg per day.

A complete clinical examination of the animals was carried out daily on weekdays from D0 to D21: weighing, taking the rectal temperature, general condition, appetite. At the weekend (period of rest without administration), the animals only underwent simple observation to check that their general condition and behaviour were normal.

Blood samples for full blood count (FBC) and biochemical assays were taken at the start and at the end of treatment (on D0 and D21). All the dogs received the whole of the treatment. No side-effects were observed when the dogs were examined.

The results of the study are as follows:
As the use of steroids may cause a weight gain, the dogs were weighed every day of the treatment. Contrary to what was expected, a weight loss was observed for all the dogs of about 4.5%.

No increase in the level of cholesterol and triglycerides was detected. On the contrary, a decrease in the quantity of these molecules was measured: -2.3% on average for cholesterol, -30% on average for triglycerides.

The use of steroids may also be the source of development of diabetes. No increase in glycaemia was observed on D21. On the contrary, a decrease in glucose of 12.9% on average was observed.

Based on the renal balance carried out (urea and creatinine), no renal toxicity was observed in the 3 dogs.

The hepatic balance (albumin, proteins, aspartate aminotransferase (AST) and alanine aminotransferase (ALT) (AST/ALT ratio), alkaline phosphatase, total bilirubin) of the 3 dogs was normal after treatment. Moreover, no significant change in the haematologic and biochemical parameters was observed.

In conclusion, the compound BIM2b administered in the form of BIM2b PO composition does not lead to any toxicity. Administration of this composition at the posology of 30 mg/ml of compound BIM2b, twice a day, 5 days per week, for 21 days did not cause undesirable clinical effects. Likewise, the parameters studied did not demonstrate hepatic or renal toxicity.

### Example 6: Study of the anti-GBM efficacy of BIM2b PO administered by the oral route to dogs with spontaneous gliomas (called "GBM dog subjects")

The purpose of this study was to evaluate the efficacy of the compound BIM2b administered in a composition according to the invention in the treatment of canine gliomas.

9 dogs with spontaneous gliomas diagnosed by imagery were included in the study. The dogs were treated orally with a daily dose of 3 mg/kg of compound BIM2b (1 ml of composition from Example 2) per 5 kg tranche.

The study comprises 5 cycles of treatment of 21 days (105 days) followed by 2 cycles of 3 months of observation. The condition and the tumour volume are evaluated by MRI.

On day D0 (1st administration), the GBM dog subject is examined clinically and a blood sample is collected for analysis of the biochemical parameters. On D15, an electrocardiogram is recorded and a blood sample is taken. Starting from D21, the dose is reviewed, either raised (+ 25% of the original dose) or lowered (-25% of the original dose) depending on the tumour response and/or toxicity.

Electrocardiograms, blood samples and MRI are carried out on D42, D63, D84 and D105, then 3 months and 6 months after stopping the treatment.

The tumour response is evaluated by MRI based on the RECIST criteria (Response Evaluation Criteria in Solid Tumours). These criteria define the tumoral response as follows:
- progression is an increase in size of the largest axis of the tumour of more than 20% relative to the preceding MRI;
- regression is defined by a decrease in the size of the largest axis of the tumour of more than 30% relative to the preceding MRI;
- stability is defined by a change in the size of the tumour between a decrease of less than 30% and an increase of less than 20%.

### Results

This study lasted 22 months. 3 of the 9 dogs included were not analysed, as they did not reach D42 (deaths due in particular to inclusion at too advanced a stage of the disease). Among the remaining 6 dogs, 2 dogs **(N** and G) died on D165 (5.5 months) and D126 (4.2 months) respectively. The tumour from GBM dog subject **N** was used for quantifying BIM2b and its metabolites (Example 8).

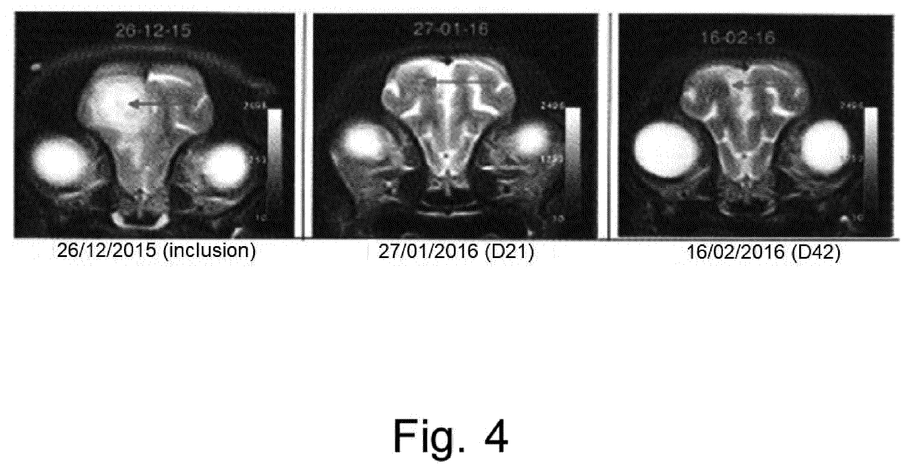

For the remaining 4 GBM dog subjects, it was found that the treatment by the oral route is well tolerated. An improvement of the clinical signs is noted for all 4 dogs: 2 remained stable and a very clear regression was observed in the other two cases almost with disappearance of the lesion for one of them **(D).** Figure 4 shows disappearance of the tumour for dog **D** at D42.

### Example 7: Analysis of BIM2b in the brains of mice treated per os with BIM2b PO

The treatment of the healthy mice per os with BIM2b PO and the provenance of the brains are described in Example 4.

The compound BIM2b and its metabolites in the brains were separated, detected and quantified by HPTLC as described in the Methods section.

The results are given in Table 1 below:

**Table 1**

| | Day | Weight of the brain (mg) | | Compound BIM2b (µg/g of brain) | |
|---|---|---|---|---|---|
| | D2 | | 415 | | 12.5 |
| | D4 | | 490 | | 7.1 |
| | D18 | | 471 | | 11.7 |
| | D22 | | 466 | | 1.0 |

The results show that the compound BIM2b is found in the brains of mice treated by the oral route with BIM2b PO in a composition according to the invention. This demonstrates that the compound BIM2b crosses the blood-brain barrier (BBB) of the treated healthy mice. On D22, an evident correlation is observed between the pronounced drop (91.5%) in the content of BIM2b in the mouse brain and stopping of the treatment on D20. The metabolites are only found as traces.

In the case of a neoplastic pathology, rather than BBB, reference is made to BTB (blood-tumour barrier). The BTB is weakened by the presence of the tumour and by its physiology. Research shows that the molecules cross the BTB more easily than the BBB.

Crossing the BBB may therefore be regarded as predictive of crossing the BTB.

The percentage of BIM2b found in the brains, relative to the total quantity that the mice ingested, is 0.013% on D18.

### Example 8: Analysis of BIM2b in a tumour of a GBM dog subject treated by the oral route with BIM2b PO.

The GBM tumour is from dog N in Example 6.

The compound BIM2b and its metabolites in the tumour are separated, detected and quantified by HPTLC as described in Section II) Methods.

The results show the presence of 3.7 µg of BIM2b in the 212.7 mg of tumour fragment analysed. The percentage by weight of BIM2b found in the whole tumour relative to the total quantity of BIM2b that the dog ingested is 0.39%; i.e. 30 times more than the percentage by weight found in the brains of healthy mice (Example 7).

The fact that necropsy of the tumour was carried out 21 days after stopping the treatment and that BIM2b is still found in the tumour shows that the compound BIM2b contained in BIM2b PO crosses the BTB and is found preferentially in the GBM tumour.

### References

1. Macala, LJ, Yu RK and Ando, S. J. Lip. Res. (1983), 24: 1243-1250.
2. Folch, J, Lees, M and Sloane-Stanley, GH. J. Biol. Chem. (1957), 226: 497-509.
3. Mann PC, Vahle J., Keenan, CM, Baker JF, Bradley, AE, Goodman, DG, Harada, T, Herbert, R, Kaufmann, W, Kellner, R, Nolte, T, Rittinghausen, S, and Tanaka, T. Toxicologic Pathology (2012), 40 (4 Suppl).

## Claims

1. Composition comprising at least one 7β-hydroxycholesterol derivative and a lipid vehicle, wherein said lipid vehicle comprises an oil or a mixture of oils, excluding a lipid vehicle consisting of lipid vesicles formed from one or more lipid layer(s) or consisting of phospholipids in the non-liposomal form, in which the 7β-hydroxycholesterol derivative corresponds to formula (I) 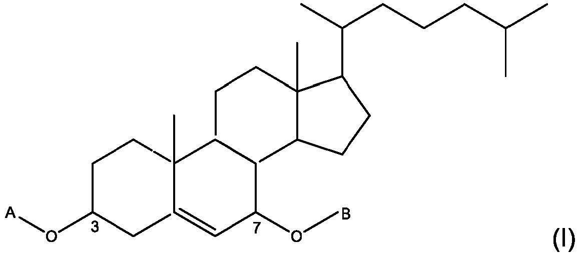 in which:
- A represents
a -C(O)R₁ group in which R₁ is a saturated heterocycle comprising 5 to 14 members and including 1 or 2 heteroatoms, unsubstituted or substituted with at least one linear or branched C₁-C₆ alkyl or a group selected from OR, NRR', NHR and SR, where R and R', independently, represent hydrogen, a linear or branched C₁-C₁₂, preferably C₁-C₆, alkyl, or an unsubstituted aryl;
or a -(R₂)ₙ- group in which R₂ is an amino acid residue bound by its C-terminal end and n= 1 to 3, each of the R₂ being identical or different, in which the N-terminal end of said amino acid may be substituted with a -C(O)R₃ group in which R₃ is a mono- or polycyclic C₆-C₁₄ arylalkyl group; a mono- or polycyclic C₅-C₁₄ heteroarylalkyl group that may comprise one or more heteroatoms, which may be identical or different; a mono- or polycyclic C₆-C₁₄ arylalkyloxy group or a mono- or polycyclic C₅-C₁₄ heteroarylalkyloxy group that may comprise one or more heteroatoms, which may be identical or different,
- B represents a -C(O)R₄ group, in which R₄ is a linear or branched C₁-C₁₂, preferably C₁-C₆, alkyl, unsubstituted or substituted with a group selected from OR, NRR', NHR and SR, as defined above; an aryl group, unsubstituted or substituted with a group selected from OR, NRR', NHR and SR, as defined above; or R₄ represents OR₅, in which R₅ is a linear or branched C₁-C₁₂, preferably C₁-C₆, alkyl.

2. Composition according to claim 1, **characterized in that** it is in a form suitable for administration by the oral route.

3. Composition according to claims 1 or 2 **characterized in that** the compound of formula (I) is in solution in said lipid vehicle.

4. Composition according to any one of claims 1 to 3, in which said lipid vehicle comprises a vegetable oil or a mixture of vegetable oils.

5. Composition according to any one of claims 1 to 4, in which said lipid vehicle comprises a vegetable oil selected from argan oil, avocado oil, linseed oil, sunflower oil, palm oil, cabbage palm oil, coconut oil, grapeseed oil, black mustard oil, poppy seed oil, shea butter oil, sweet almond oil, soya oil, peanut oil, cottonseed oil, sesame oil, olive oil, maize oil, cocoa oil, castor oil, Moringa oil (or Ben oil), rapeseed oil, annatto oil, wheatgerm oil, safflower oil, walnut oil, hazelnut oil, turnip rape oil or mixtures thereof.

6. Composition according to any one of claims 1 to 5, in which said lipid vehicle is argan oil.

7. Composition according to any one of claims 1 to 6, **characterized in that**, in formula (I), A represents a -C(O)R₁ group in which R₁ is a saturated heterocycle comprising 5 members and including 2 oxygen atoms, substituted with at least one linear or branched C₁-C₆ alkyl.

8. Composition according to any one of claims 1 to 7, **characterized in that**, in formula (I), B represents a -C(O)R₄ group in which R₄ is a linear or branched C₁-C₆ alkyl, or R₄ represents OR₅, in which R₅ is a linear or branched C₁-C₁₂, preferably C₁-C₆, alkyl.

9. Composition according to any one of claims 1 to 8, **characterized in that**, in formula (I), B represents an acyl group in which the alkyl group is C₁-C₆ or an alkoxycarbonyl group in which the alkyl group is C₁-C₆.

10. Composition according to claim 1 to 6, 8 or 9, **characterized in that**, in formula (I), A represents:
- a -(R₂)ₙ- group in which R₂ is an amino acid residue and n = 2; or
- a -(R₂)ₙ- group in which R₂ is an amino acid residue, n= 2 and the N-terminal end of said amino acid is substituted with an arylalkoxycarbonyl group, in particular benzyloxycarbonyl; or
- an alanyl radical bound to a glycinyl radical, optionally substituted at its N-terminal end with an arylalkoxycarbonyl group, in particular benzyloxycarbonyl; or
- a methionyl radical bound to a glycinyl radical, optionally substituted at its N-terminal end with an arylalkoxycarbonyl group, in particular benzyloxycarbonyl, and
- B is as defined in any one of claims 1, 8 or 9.

11. Composition according to any one of claims 1 to 10, in which the 7β-hydroxycholesterol derivative is selected from the compounds of formula (I) in which:
- A is a 3-benzyloxycarbonyl-glycinyl-alanyl group and B is a tert-butoxycarbonyl group, or
- A is a 3-benzyloxycarbonyl-glycinyl-alanyl group and B is an acetyl group, or
- A is a -C(O)R₁ group in which R₁ is a 2,2-dimethyl-1,3-dioxolane group and B is a tert-butoxycarbonyl group.

12. Composition according to any one of claims 1 to 9 or 11, in which the 7β-hydroxycholesterol derivative is a compound of formula (I) in which A is a -C(O)R₁ group in which R₁ is a 2,2-dimethyl-1,3-dioxolane group and B is an acetyl group, of formula

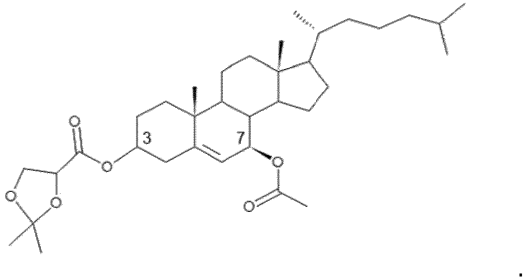

13. Composition according to any one of claims 1 to 12, in which the content of compound of formula (I) is from 0.1 to 3.5% (w/v) of the total volume of the composition.

14. Composition according to any one of claims 1 to 13, **characterized in that** the content of compound of formula (I) is from 1 to 35 mg/ml, preferably 30 mg/ml.

15. Composition according to any one of claims 1 to 14, **characterized in that** the content of lipid vehicle is from 90 to 99% (v/v) of the total volume of the composition.

16. Pharmaceutical composition comprising a composition according to any one of claims 1 to 15.

17. Pharmaceutical composition according to claim 16, **characterized in that** it is in a form suitable for administration by the oral route.

18. Method for preparing a composition according to any one of claims 1 to 13, comprising the following steps:
- preparing a mixture containing the 7β-hydroxycholesterol derivative of formula (I) as defined in any one of claims 1 or 9 to 12, a lipid vehicle and a co-solvent,
- optionnally adding an antioxidant and
- if applicable, evaporating the co-solvent.

19. Method according to claim 18, wherein the lipid vehicle is as defined in any one of claims 4 to 6.

20. Composition according to any one of claims 1 to 17, for use in the treatment of a neoplastic pathology or a malignant haemopathy.

21. Composition for use according to claim 20, in which the neoplastic pathology is glioblastoma multiforme.

## Patentansprüche

1. Zusammensetzung, umfassend mindestens ein 7β-Hydroxycholesterinderivat und ein Lipidvehikel, wobei das Lipidvehikel ein Öl oder eine Mischung von Ölen umfasst, wobei ein Lipidvehikel ausgeschlossen ist, das aus Lipidvesikeln besteht, die aus einer oder mehreren Lipidschicht(en) gebildet sind oder aus Phospholipiden in der nicht liposomalen Form bestehen, wobei das 7β-Hydroxycholesterinderivat der Formel (I) entspricht 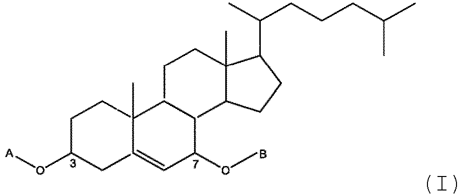 wobei:
- A
eine -C(O)R₁-Gruppe darstellt, wobei R₁ ein gesättigter Heterocyclus ist, umfassend 5 bis 14 Elemente und einschließend 1 oder 2 Heteroatome, unsubstituiert oder substituiert mit mindestens einem linearen oder verzweigten C₁-C₆-Alkyl oder einer Gruppe, ausgewählt aus OR, NRR', NHR und SR, wobei R und R' unabhängig voneinander Wasserstoff, ein lineares oder verzweigtes C₁-C₁₂-, vorzugsweise C₁-C₆-Alkyl, oder ein unsubstituiertes Aryl darstellen;
oder eine - (R₂)ₙ-Gruppe darstellt, wobei R₂ ein Aminosäurerest ist, der durch sein C-terminales Ende gebunden ist, und n = 1 bis 3, wobei jedes der R₂ gleich oder verschieden sein kann, wobei das N-terminale Ende der genannten Aminosäure durch eine -C(O)R₃-Gruppe ersetzt sein kann, in der R₃ eine mono- oder polycyclische C₆-C₁₄-Arylalkylgruppe ist; eine mono- oder polycyclische C₅-C₁₄-Heteroarylalkylgruppe ist, die ein oder mehrere Heteroatome umfassen kann, die gleich oder verschieden sein können; eine mono- oder polycyclische C₆-C₁₄-Arylalkyloxygruppe ist oder eine mono- oder polycyclische C₅-C₁₄-Heteroarylalkyloxygruppe ist, die ein oder mehrere Heteroatome umfassen kann, die gleich oder verschieden sein können,
- B eine -C(O)R₄-Gruppe darstellt, wobei R₄ eine lineare oder verzweigte C₁-C₁₂-, vorzugsweise C₁-C₆-Alkylgruppe, die unsubstituiert oder substituiert ist mit einer Gruppe ausgewählt aus OR, NRR', NHR und SR, wie oberhalb definiert; eine Arylgruppe, die unsubstituiert oder substituiert ist mit einer Gruppe ausgewählt aus OR, NRR', NHR und SR, wie oberhalb definiert; oder R₄ OR₅ darstellt, wobei R₅ eine lineare oder verzweigte C₁-C₁₂-, vorzugsweise C₁-C₆-Alkylgruppe, ist.

2. Zusammensetzung nach Anspruch 1, **dadurch gekennzeichnet, dass** sie in einer Form vorliegt, die für die orale Verabreichung geeignet ist.

3. Zusammensetzung nach Anspruch 1 oder 2, **dadurch gekennzeichnet, dass** die Verbindung der Formel (I) in Lösung in dem Lipidvehikel vorliegt.

4. Zusammensetzung nach einem der Ansprüche 1 bis 3, wobei das Lipidvehikel ein Pflanzenöl oder eine Mischung von Pflanzenölen umfasst.

5. Zusammensetzung nach einem der Ansprüche 1 bis 4, wobei das Lipidvehikel ein Pflanzenöl umfasst, das ausgewählt ist aus Arganöl, Avocadoöl, Leinöl, Sonnenblumenöl, Palmöl, Kohlpalmenöl, Kokosnussöl, Traubenkernöl, schwarzem Senföl, Mohnöl, Sheabutteröl, Süßmandelöl, Sojaöl, Erdnussöl, Baumwollsamenöl, Sesamöl, Olivenöl, Maisöl, Kakaoöl, Rizinusöl, Moringaöl (oder Ben-Öl), Rapsöl, Annattoöl, Weizenkeimöl, Safloröl, Walnussöl, Haselnussöl, Rüböl oder Mischungen davon.

6. Zusammensetzung nach einem der Ansprüche 1 bis 5, wobei das Lipidvehikel Arganöl ist.

7. Zusammensetzung nach einem der Ansprüche 1 bis 6, **dadurch gekennzeichnet, dass** in der Formel (I) A eine-C(O)R₁-Gruppe darstellt, wobei R₁ ein gesättigter Heterocyclus ist, umfassend 5 Elemente und einschließend 2 Sauerstoffatome, substituiert mit mindestens einem linearen oder verzweigten C₁-C₆-Alkyl.

8. Zusammensetzung nach einem der Ansprüche 1 bis 7, **dadurch gekennzeichnet, dass** in der Formel (I) B eine-C(O)R₄-Gruppe darstellt, wobei R₄ ein lineares oder verzweigtes C₁-C₆-Alkyl ist, oder R4 OR₅ darstellt, wobei R₅ ein lineares oder verzweigtes C₁-C₁₂-, vorzugsweise C₁-C₆-Alkyl ist.

9. Zusammensetzung nach einem der Ansprüche 1 bis 8, **dadurch gekennzeichnet, dass** in der Formel (I) B eine Acylgruppe darstellt, wobei die Alkylgruppe C₁-C₆ ist, oder eine Alkoxycarbonylgruppe, wobei die Alkylgruppe C₁-C₆ ist.

10. Zusammensetzung nach Anspruch 1 bis 6, 8 oder 9, **dadurch gekennzeichnet, dass** in Formel (I) A Folgendes darstellt:
- eine -(R₂)ₙ-Gruppe, wobei R₂ ein Aminosäurerest ist und n = 2; oder
- eine -(R₂)ₙ-Gruppe, wobei R₂ ein Aminosäurerest ist, n = 2 und das N-terminale Ende der Aminosäure mit einer Arylalkoxycarbonylgruppe, insbesondere Benzyloxycarbonyl, substituiert ist; oder
- ein Alanylradikal, gebunden an ein Glycinylradikal, optional substituiert an seinem N-terminalen Ende mit einer Arylalkoxycarbonylgruppe, insbesondere Benzyloxycarbonyl; oder
- ein Methionylradikal, gebunden an ein Glycinylradikal, optional substituiert an seinem N-terminalen Ende mit einer Arylalkoxycarbonylgruppe, insbesondere Benzyloxycarbonyl; und
- B wie in einem der Ansprüche 1, 8 oder 9 definiert ist.

11. Zusammensetzung nach einem der Ansprüche 1 bis 10, wobei das 7β-Hydroxycholesterinderivat aus den Verbindungen der Formel (I) ausgewählt ist, wobei:
- A eine 3-Benzyloxycarbonyl-Glycinyl-Alanylgruppe ist und B eine tert-Butoxycarbonylgruppe ist, oder
- A eine 3-Benzyloxycarbonyl-Glycinyl-Alanylgruppe und B eine Acetylgruppe ist, oder
- A eine -C(O)R₁-Gruppe ist, wobei R₁ eine 2,2-Dimethyl-1,3-dioxolan-Gruppe ist und B eine tert-Butoxycarbonylgruppe ist.

12. Zusammensetzung nach einem der Ansprüche 1 bis 9 oder 11, wobei das 7β-Hydroxycholesterinderivat eine Verbindung der Formel (I) ist, wobei A eine -C(O)R₁-Gruppe ist, wobei R₁ eine 2,2-Dimethyl-1,3-dioxolangruppe ist und B eine Acetylgruppe ist, der Formel

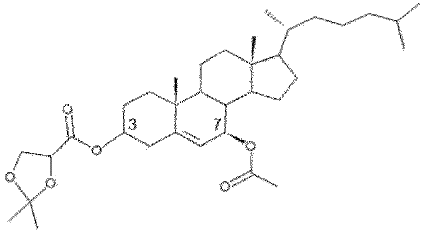

13. Zusammensetzung nach einem der Ansprüche 1 bis 12, wobei der Inhalt der Verbindung der Formel (I) 0,1 bis 3,5 % (Gew./Vol.) des Gesamtvolumens der Zusammensetzung beträgt.

14. Zusammensetzung nach einem der Ansprüche 1 bis 13, **dadurch gekennzeichnet, dass** der Inhalt der Verbindung der Formel (I) 1 bis 35 mg/ml, vorzugsweise 30 mg/ml, beträgt.

15. Zusammensetzung nach einem der Ansprüche 1 bis 14, **dadurch gekennzeichnet, dass** der Inhalt des Lipidvehikels 90 bis 99 % (Vol./Vol.) des Gesamtvolumens der Zusammensetzung beträgt.

16. Pharmazeutische Zusammensetzung, umfassend eine Zusammensetzung nach einem der Ansprüche 1 bis 15.

17. Pharmazeutische Zusammensetzung nach Anspruch 16, **dadurch gekennzeichnet, dass** sie in einer Form vorliegt, die für die orale Verabreichung geeignet ist.

18. Verfahren zum Herstellen einer Zusammensetzung nach einem der Ansprüche 1 bis 13, umfassend die folgenden Schritte:
- Zubereiten einer Mischung, die das 7β-Hydroxycholesterinderivat der Formel (I), wie in einem der Ansprüche 1 oder 9 bis 12 definiert, ein Lipidvehikel und ein Hilfslösungsmittel enthält,
- optionales Hinzufügen eines Antioxidationsmittels und
- falls anwendbar, Verdampfen des Hilfslösungsmittels.

19. Verfahren nach Anspruch 18, wobei das Lipidvehikel nach einem der Ansprüche 4 bis 6 definiert ist.

20. Zusammensetzung nach einem der Ansprüche 1 bis 17, zur Verwendung bei der Behandlung einer neoplastischen Pathologie oder einer malignen Hämopathie.

21. Zusammensetzung zur Verwendung nach Anspruch 20, wobei die neoplastische Pathologie Glioblastoma multiforme ist.

## Revendications

1. Composition comprenant au moins un dérivé de 7β-hydroxycholestérol et un véhicule lipidique, dans laquelle ledit véhicule lipidique comprend une huile ou un mélange d'huiles, à l'exclusion d'un véhicule lipidique consistant en des vésicules lipidiques formées d'une ou de plusieurs couches lipidiques ou consistant en des phospholipides sous forme non liposomale, dans laquelle le dérivé de 7β-hydroxycholestérol correspond à la formule (I) 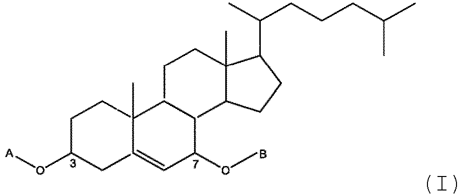 dans laquelle :
- A représente
un groupe -C(O)R₁ dans lequel R₁ est un hétérocycle saturé comprenant 5 à 14 chaînons et incluant 1 ou 2 hétéroatomes, non substitué ou substitué avec au moins un alkyle linéaire ou ramifié en C₁-C₆ ou un groupe sélectionné parmi OR, NRR', NHR et SR, où R et R', indépendamment, représentent un hydrogène, un alkyle linéaire ou ramifié en C₁-C₁₂, de préférence en C₁-C₆, ou un aryle non substitué ;
ou un groupe -(R₂)ₙ- dans lequel R₂ est un résidu d'acide aminé lié par son extrémité C-terminale et n = 1 à 3, chacun des R₂ étant identique ou différent, dans lequel l'extrémité N-terminale dudit acide aminé peut être substitué avec un groupe -C(O)R₃ dans lequel R₃ est un groupe arylalkyle mono- ou poly-cyclique en C₆-C₁₄ ; un groupe hétéroarylalkyle mono- ou poly-cyclique en C₅-C₁₄ qui peut comprendre un ou plusieurs hétéroatomes, qui peuvent être identiques ou différents ; un groupe arylalkyloxy mono- ou poly-cyclique en C₆-C₁₄ ou un groupe hétéroarylalkyloxy mono- ou poly-cyclique en C₅-C₁₄ qui peut comprendre un ou plusieurs hétéroatomes, qui peuvent être identiques ou différents,
- B représente un groupe -C(O)R₄, dans lequel R₄ est un alkyle linéaire ou ramifié en C₁-C₁₂, de préférence en C₁-C₆, non substitué ou substitué avec un groupe sélectionné parmi OR, NRR', NHR et SR, tel que défini ci-dessus ; un groupe aryle, non substitué ou substitué avec un groupe sélectionné parmi OR, NRR', NHR et SR, tel que défini ci-dessus ; ou R₄ représente OR₅, dans lequel R₅ est un alkyle linéaire ou ramifié en C₁-C₁₂, de préférence en C₁-C₆.

2. Composition selon la revendication 1, **caractérisée en ce qu'**elle est sous une forme appropriée pour une administration par voie orale.

3. Composition selon les revendications 1 ou 2, **caractérisée en ce que** le composé de formule (I) est en solution dans ledit véhicule lipidique.

4. Composition selon l'une quelconque des revendications 1 à 3, dans laquelle ledit véhicule lipidique comprend une huile végétale ou un mélange d'huiles végétales.

5. Composition selon l'une quelconque des revendications 1 à 4, dans laquelle ledit véhicule lipidique comprend une huile végétale sélectionnée parmi l'huile d'argan, l'huile d'avocat, l'huile de lin, l'huile de tournesol, l'huile de palme, l'huile de chou palmiste, l'huile de noix de coco, l'huile de pépins de raisin, l'huile de moutarde noire, l'huile de pépins de pavot, l'huile de beurre de karité, l'huile d'amande douce, l'huile de soja, l'huile d'arachide, l'huile de graines de coton, l'huile de sésame, l'huile d'olive, l'huile de maïs, l'huile de cacao, l'huile de ricin, l'huile de moringa (ou huile de ben), l'huile de colza, l'huile de rocou, l'huile de germe de blé, l'huile de carthame, l'huile de noix, l'huile de noisette, l'huile de navette, ou les mélanges de celles-ci.

6. Composition selon l'une quelconque des revendications 1 à 5, dans laquelle ledit véhicule lipidique est l'huile d'argan.

7. Composition selon l'une quelconque des revendications 1 à 6, **caractérisée en ce que**, dans la formule (I), A représente un groupe -C(O)R₁ dans lequel R₁ est un hétérocycle saturé comprenant 5 chaînons et incluant 2 atomes d'oxygène, substitué avec au moins un alkyle linéaire ou ramifié en C₁-C₆.

8. Composition selon l'une quelconque des revendications 1 à 7, **caractérisée en ce que**, dans la formule (I), B représente un groupe -C(O)R₄ dans lequel R₄ est un alkyle linéaire ou ramifié en C₁-C₆, ou R₄ représente OR₅, dans lequel R₅ est un alkyle linéaire ou ramifié en C₁-C₁₂, de préférence en C₁-C₆.

9. Composition selon l'une quelconque des revendications 1 à 8, **caractérisée en ce que**, dans la formule (I), B représente un groupe acyle dans lequel le groupe alkyle est en C₁-C₆ ou un groupe alcoxycarbonyle dans lequel le groupe alkyle est en C₁-C₆.

10. Composition selon les revendications 1 à 6, 8 ou 9, **caractérisée en ce que**, dans la formule (I), A représente :
- un groupe -(R₂)ₙ- dans lequel R₂ est un résidu d'acide aminé et n = 2 ; ou
- un groupe -(R₂)ₙ- dans lequel R₂ est un résidu d'acide aminé, n = 2 et l'extrémité N-terminale dudit acide aminé est substituée avec un groupe arylalcoxycarbonyle, en particulier un benzyloxycarbonyle ; ou
- un radical alanyle lié à un radical glycinyle, facultativement substitué à son extrémité N-terminale avec un groupe arylalcoxycarbonyle, en particulier un benzyloxycarbonyle ; ou
- un radical méthionyle lié à un radical glycinyle, facultativement substitué à son extrémité N-terminale avec un groupe arylalcoxycarbonyle, en particulier un benzyloxycarbonyle, et
- B est tel que défini dans l'une quelconque des revendications 1, 8 ou 9.

11. Composition selon l'une quelconque des revendications 1 à 10, dans laquelle le dérivé de 7β-hydroxycholestérol est sélectionné parmi les composés de formule (I) dans laquelle :
- A est un groupe 3-benzyloxycarbonyl-glycinyl-alanyle et B est un groupe tert-butoxycarbonyle, ou
- A est un groupe 3-benzyloxycarbonyl-glycinyl-alanyle et B est un groupe acétyle, ou
- A est un groupe -C(O)R₁ dans lequel R₁ est un groupe 2,2-diméthyl-1,3-dioxolane et B est un groupe tert-butoxycarbonyle.

12. Composition selon l'une quelconque des revendications 1 à 9 ou 11, dans laquelle le dérivé de 7β-hydroxycholestérol est un composé de formule (I) dans laquelle A est un groupe -C(O)R₁ dans lequel R₁ est un groupe 2,2-diméthyl-1,3-dioxolane et B est un groupe acétyle, de formule

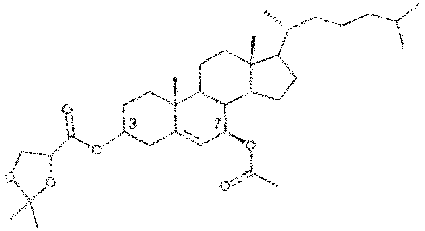

13. Composition selon l'une quelconque des revendications 1 à 12, dans laquelle la teneur en composé de formule (I) est de 0,1 à 3,5 % (en poids/volume) du volume total de la composition.

14. Composition selon l'une quelconque des revendications 1 à 13, **caractérisée en ce que** la teneur en composé de formule (I) est de 1 à 35 mg/ml, de préférence de 30 mg/ml.

15. Composition selon l'une quelconque des revendications 1 à 14, **caractérisée en ce que** la teneur en véhicule lipidique est de 90 à 99 % (en volume/volume) du volume total de la composition.

16. Composition pharmaceutique comprenant une composition selon l'une quelconque des revendications 1 à 15.

17. Composition pharmaceutique selon la revendication 16, **caractérisée en ce qu'**elle est sous une forme appropriée pour une administration par voie orale.

18. Procédé de préparation d'une composition selon l'une quelconque des revendications 1 à 13, comprenant les étapes suivantes :
- la préparation d'un mélange contenant le dérivé de 7β-hydroxycholestérol de formule (I) tel que défini dans l'une quelconque des revendications 1 ou 9 à 12, un véhicule lipidique et un co-solvant,
- l'ajout facultatif d'un antioxydant et
- le cas échéant, l'évaporation du co-solvant.

19. Procédé selon la revendication 18, dans lequel le véhicule lipidique est tel que défini dans l'une quelconque des revendications 4 à 6.

20. Composition selon l'une quelconque des revendications 1 à 17, pour une utilisation dans le traitement d'une pathologie néoplasique ou d'une hémopathie maligne.

21. Composition pour une utilisation selon la revendication 20, dans laquelle la pathologie néoplasique est un glioblastome multiforme.
